# EUROPEAN PATENT APPLICATION

(11) **EP 3 760 203 A1**
(43) Date of publication of application: **06.01.2021**
(21) Application number: 19184227.7
(22) Date of filing: 03.07.2019
(51) Int. Cl.: A61K 31/496, A61K 31/122, A61K 31/575, A61K 38/46, A61K 45/06, A61K 9/00, A61P 3/00, A61P 43/00

(54) **MEDICAMENT FOR PREVENTION OR TREATMENT OF A METABOLIC DISORDER**

(71) Applicant: Medizinische Universität Graz, 8036 Graz (AT)
(72) Inventor: WAGNER, Martin, 8010 Graz (AT); PANZITT, Katrin, 8010 Graz (AT)
(74) Representative: Sonn & Partner Patentanwälte

(57) **Abstract**

The present invention provides a pharmaceutical composition for use in prevention or treatment of a metabolic disorder selected from alpha-1 antitrypsin deficiency (A1AD) and lysosomal storage disease. The composition comprises a pregnane X receptor (PXR) ligand selected from the group consisting of rifampicin, hyper-forin and ursodeoxycholic acid (UDCA). Furthermore, pharmaceutical compositions, sets and an inhalation device suitable for treating said metabolic disorder are provided.

## Description

The present invention relates to pharmaceutical compositions for use in prevention or treatment of metabolic, hepatic storage diseases selected from alpha-1 antitrypsin deficiency (A1AD) and lysosomal storage disease.

### Background

Alpha-1 antitrypsin deficiency (A1AD) is a metabolic disorder typically caused by mutations in the Serpin peptidase inhibitor, clade A, member 1 (SERPINA1) gene and usually characterized by low serum levels of alpha-1 antitrypsin (A1AT; see e.g. Fregonese, Laura, and Jan Stolk. "Hereditary alpha-1-antitrypsin deficiency and its clinical consequences." Orphanet journal of rare diseases 3.1 (2008): 16). Clinical manifestations usually include potentially fatal liver cirrhosis and pulmonary emphysema.

Diagnosis typically involves measuring A1AT serum levels, A1AT protein phenotyping e.g. by protein electrophoresis and isoelectric focusing and/or A1AT genotyping (see e.g. Aboussouan, Loutfi S., and James K. Stoller. "Detection of alpha-1 antitrypsin deficiency: a review." Respiratory medicine 103.3 (2009): 335-341).

A1AT is produced primarily in hepatocytes and released into the blood circulation by the liver. One of the protein's functions is to protect the alveolar tissue of the lungs from proteolytic damage, primarily from neutrophil elastase. Mutant A1AT may polymerize within the hepatocyte, precluding secretion into the blood and causing low serum A1AT levels (see e.g. Stoller, James K., and Loutfi S. Aboussouan. "A review of α1-antitrypsin deficiency." American journal of respiratory and critical care medicine 185.3 (2012): 246-259). This can lead to retention of aggregates of A1AT in hepatocytes, ultimately leading to liver cirrhosis.

Treatment options for A1AD are limited. For the treatment of lung disease associated with A1AD, bronchodilators and inhaled corticosteroids may be used. Moreover, intravenous augmentation therapy may be used, in which pooled human plasma A1AT is infused to raise serum A1AT levels above the protective threshold value. However, this approach is costly, requires frequent injections over a subject's lifetime and is only partially effective for treating lung disease associated with the disorder. It does not prevent or treat A1AD-associated liver disease, which in severe cases may require liver transplantation.

Lysosomal storage diseases (LSDs) constitute another case of inherited metabolic disorders. They result from defects in lysosomal function. They are a group of at least 50 genetic diseases, each one typically resulting from a deficiency of a particular lysosomal protein/activity or from non-lysosomal activities e.g. involved in lysosomal biogenesis (see e.g. Fuller, Maria, Peter J. Meikle, and John J. Hopwood. "Epidemiology of lysosomal storage diseases: an overview." Fabry disease: perspectives from 5 years of FOS. Oxford PharmaGenesis, 2006). Although LSDs result from mutations in different genes, they generally share the biochemical characteristic of resulting in an accumulation of substrates within lysosomes.

Treatment options for LSDs are limited. Generally there are no cures and treatment is mostly symptomatic. Bone marrow transplantation and enzyme replacement therapy have been tried in some cases.

Lysosomal acid lipase (LAL)-deficiency is a LSD typically characterized by the body not producing enough active LAL because of mutations of the LIPA gene. This can lead to a diminished breakdown of cholesteryl esters and triglycerides, which can build up in the liver, spleen, and other organs. Clinical manifestations usually include progressive liver fibrosis and cirrhosis. LAL-deficiency can be treated with sebelipase alfa, which is a recombinant form of LAL (see e.g. Frampton, James E. "Sebelipase alfa: a review in lysosomal acid lipase deficiency." American Journal of Cardiovascular Drugs 16.6 (2016): 461-468). LAL-deficiency was historically referred to as two separate disorders: Wolman disease and Cholesteryl Ester Storage Disease (CESD). CESD is typically a milder form of LAL-deficiency than Woman disease since residual activity of LAL may still be present. However, today both Wolman disease and CESD are known as LAL-deficiency, as both are due to a deficiency of LAL (see e.g. Reiner, Željko, et al. "Lysosomal acid lipase deficiency-an under-recognized cause of dyslipidaemia and liver dysfunction." Atherosclerosis 235.1 (2014): 21-30). Also in the context of the present invention, the term "LAL-deficiency" encompasses both Wolman disease and CESD.

It is an object of the present invention to provide new and, in particular, more effective options for prevention or treatment of metabolic disorders such as A1AD and LSDs.

### Summary of the invention

Therefore, the present invention provides a pharmaceutical composition for use in prevention or treatment of a metabolic disorder selected from alpha-1 antitrypsin deficiency (A1AD) and lysosomal storage disease. The composition comprises a pregnane X receptor (PXR) ligand selected from the group consisting of rifampicin, hyperforin and ursodeoxycholic acid (UDCA).

In another aspect, the present invention relates to a set comprising a first pharmaceutical composition comprising a PXR ligand selected from the group consisting of rifampicin, hyperforin and UDCA, and a second pharmaceutical composition comprising A1AT.

In a further aspect, the present invention provides a set comprising a first pharmaceutical composition comprising a PXR ligand selected from the group consisting of rifampicin, hyperforin and UDCA, and a second pharmaceutical composition comprising a bronchodilator and/or a steroid.

A further aspect of the present invention relates to a pharmaceutical composition comprising A1AT and a PXR ligand selected from the group consisting of rifampicin, hyperforin and UDCA, preferably wherein the pharmaceutical composition is for intravenous administration, preferably wherein the pharmaceutical composition is a powder for reconstitution (e.g. in a physiologic salt solution) or as a solution (e.g. an isotonic solution with physiological pH).

Yet another aspect of the present invention relates to a pharmaceutical composition comprising an active agent selected from the group consisting of bronchodilators and steroids, and a PXR ligand selected from the group consisting of rifampicin, hyperforin and UDCA, preferably wherein the pharmaceutical composition is for inhalation, preferably wherein the pharmaceutical composition is a powder, a solution or an aerosol.

In a further aspect, the present invention provides an inhalation device for administration of a pharmaceutical composition, wherein the device comprises the pharmaceutical composition, wherein the composition comprises an active agent selected from the group consisting of bronchodilators and steroids, and a PXR ligand selected from the group consisting of rifampicin, hyperforin and UDCA; preferably wherein the inhalation device is a metered-dose inhaler, a dry-powder inhaler or a nebuliser, and/or preferably wherein the pharmaceutical composition is a pharmaceutical composition according to the present invention.

The present invention further provides a set comprising a first pharmaceutical composition comprising a PXR ligand selected from the group consisting of rifampicin, hyperforin and UDCA, and a second pharmaceutical composition comprising sebelipase alfa.

In addition, the present invention provides a pharmaceutical composition comprising sebelipase alfa and a PXR ligand selected from the group consisting of rifampicin, hyperforin and UDCA, preferably wherein the pharmaceutical composition is for intravenous administration, preferably wherein the pharmaceutical composition is a powder for reconstitution (e.g. in a physiologic salt solution) or as a solution (e.g. an isotonic solution with physiological pH).

In yet another aspect, the present invention provides a method for delaying the onset of or treating a metabolic disorder selected from A1AD and lysosomal storage disease, comprising
- obtaining a pharmaceutically acceptable formulation comprising a PXR ligand selected from rifampicin, hyperforin and UDCA; and
- administering an effective amount of the formulation to an individual having said metabolic disorder or at risk of developing said metabolic disorder.

All embodiments of the invention are described together in the following detailed description and all preferred embodiments relate to all embodiments and aspects alike. All detailed descriptions, e.g. of compositions or modes of administration, relate to preferred embodiments of all aspects of the invention, e.g. compositions for use, sets and methods. All embodiments can be combined with each other, except where stated otherwise.

### Detailed description

The present invention is based on the surprising discovery of a novel signalling pathway for regulating autophagy. It was found that the pregnane X receptor (PXR), also known as the steroid and xenobiotic sensing nuclear receptor or nuclear receptor subfamily 1, group I, member 2, can stimulate autophagy both directly and via the lyosomal biogenesis factor transcription factor EB (TFEB). Stimulating autophagy can enhance the elimination of intracellular aggregates, making PXR an attractive new drug target for disorders such as A1AD and LSD. In addition, its newly found interaction with TFEB, a master gene for lysosomal biogenesis, is an additional reason why PXR is an attractive new drug target for LSDs.

Autophagy is a cellular process which is one of the main mechanisms for the clearance of accumulated misfolded proteins. It has therefore been investigated extensively as a therapeutic target for the treatment of disorders involving protein accumulation (see e.g. Metcalf, Daniel J., et al. "Autophagy and misfolded proteins in neurodegeneration." Experimental neurology 238.1 (2012): 22-28). However, since the regulation of autophagy is very complex, there has been a lack of suitable drug targets.

PXR is a transcription factor that has a role in sensing the presence of foreign toxic substances and in regulating their metabolism. Several agonists of PXR are known and clinically tested, including rifampicin, hyperforin and ursodeoxycholic acid (UDCA). In the course of the present invention it has been surprisingly found that PXR can stimulate autophagy, making known PXR ligands a new treatment option for diseases involving intracellular aggregates, such as A1AD and LSD.

Rifampicin (see e.g. Howard, Paul, et al. "Rifampin (INN rifampicin)." Journal of pain and symptom management 50.6 (2015): 891-895), also known as rifampin, is an antibiotic that has been widely used for a long time to treat several types of bacterial infections. Hyperforin (see e.g. Beerhues, Ludger. "Hyperforin." Phytochemistry 67.20 (2006): 2201-2207) is a phytochemical produced e.g. by St John's wort (Hypericum perforatum), which can also be synthesized chemically (Sparling, Brian A., David C. Moebius, and Matthew D. Shair. "Enantioselective total synthesis of hyperforin." Journal of the American Chemical Society 135.2 (2012): 644-647). It has been proposed that hyperforin may be an active agent responsible for the antidepressant and anxiolytic properties of the extracts of St John's wort. UDCA, also known as ursodiol, is a bile acid with several medical uses (see e.g. Iser, J. H., and A. Sali. "Chenodeoxycholic acid: a review of its pharmacological properties and therapeutic use." Drugs 21.2 (1981): 90-119).

Enhancing autophagy has been proposed for the treatment of A1AD in the past (see e.g. Perlmutter, D. H. "Autophagic disposal of the aggregation-prone protein that causes liver inflammation and carcinogenesis in α-1-antitrypsin deficiency." Cell death and differentiation 16.1 (2009): 39); however, there is a lack of suitable therapeutic agents. Altogether independently from autophagy and PXR, UDCA has previously been investigated as a potential treatment option for A1AD (Lykavieris, Panayotis, et al. "Liver disease associated with ZZ α1-antitrypsin deficiency and ursodeoxycholic acid therapy in children." Journal of pediatric gastroenterology and nutrition 47.5 (2008): 623-629). However, prior to the present invention it was unknown that PXR agonists stimulate autophagy and are therefore useful for the treatment of A1AD.

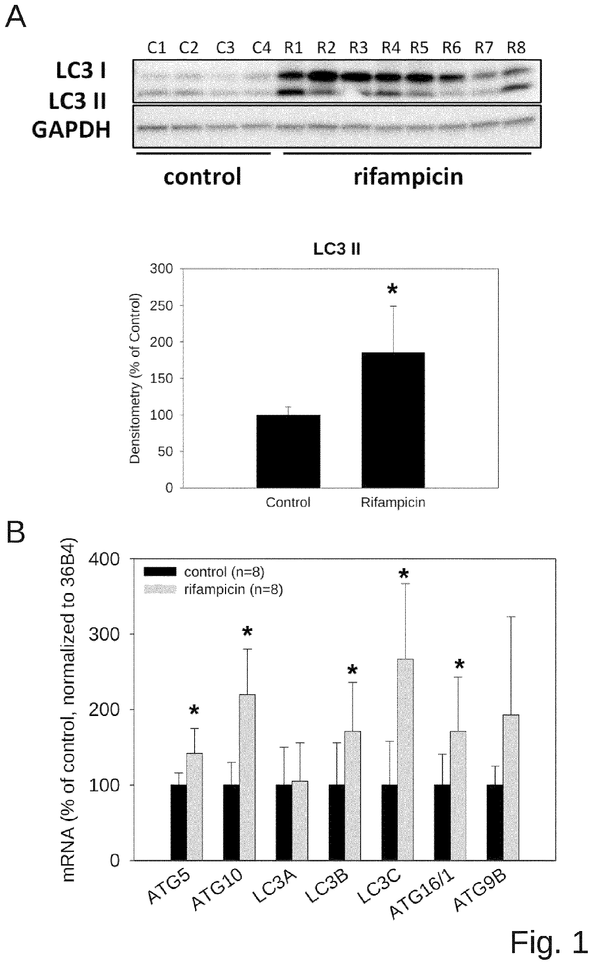

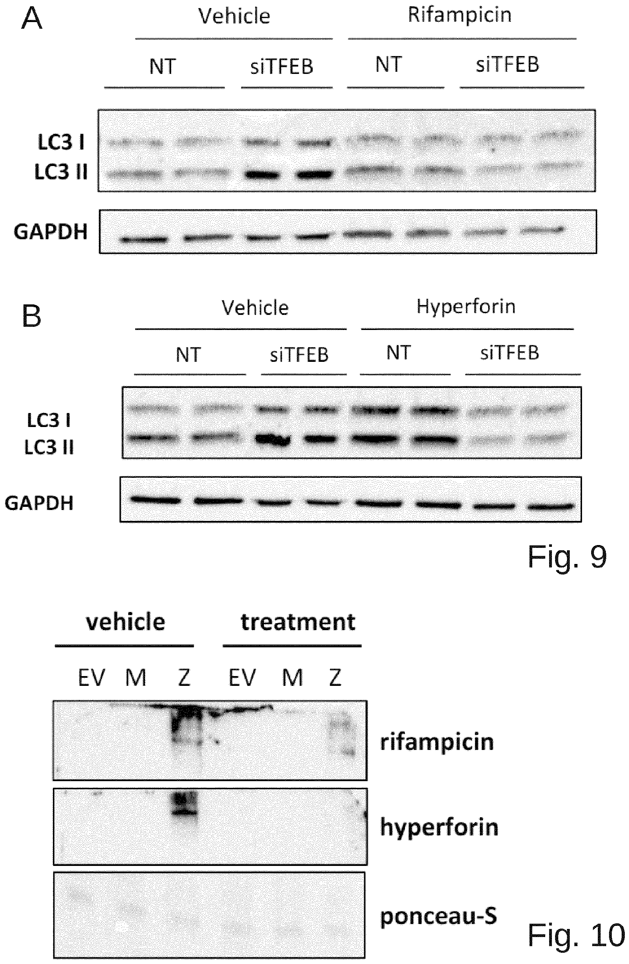

In the course of the present invention, it has been found that PXR ligands such as rifampicin, hyperforin and UDCA stimulate autophagy in the human liver as well as in isolated liver cell lines (see Example 1 and Figures 1 - 6 and 10). Strikingly, it has in addition been demonstrated that these ligands reduce accumulated A1AT in liver cells, which is the cause for A1AD-associated liver disease (see Example 1 and Figure 10). PXR ligands are therefore well suited for the treatment of A1AD.

TFEB is a master gene for lysosomal biogenesis. It encodes a transcription factor that coordinates the expression of a number of autophagy and lysosomal genes. TFEB has been proposed as a therapeutic target for LSDs (see e.g. Spampanato, Carmine, et al. "Transcription factor EB (TFEB) is a new therapeutic target for Pompe disease." EMBO molecular medicine 5.5 (2013): 691-706). Altogether independently from the newly found pathway involving PXR and TFEB, the use of rifampicin in Gaucher disease (Alisky, Joseph Martin. "Doxycycline and rifampin could be useful therapeutic agents for Gaucher disease." Molecular genetics and metabolism 90.1 (2007): 112) and the use of UDCA in Niemann-Pick disease (Evans, William RH, et al. "Case Report: Ursodeoxycholic acid treatment in Niemann-Pick disease type C; clinical experience in four cases." Wellcome open research 2 (2017)) have previously been investigated. However, prior to the present invention it was not known that TFEB could be stimulated through PXR and that PXR agonists could therefore be used for the treatment of LSDs.

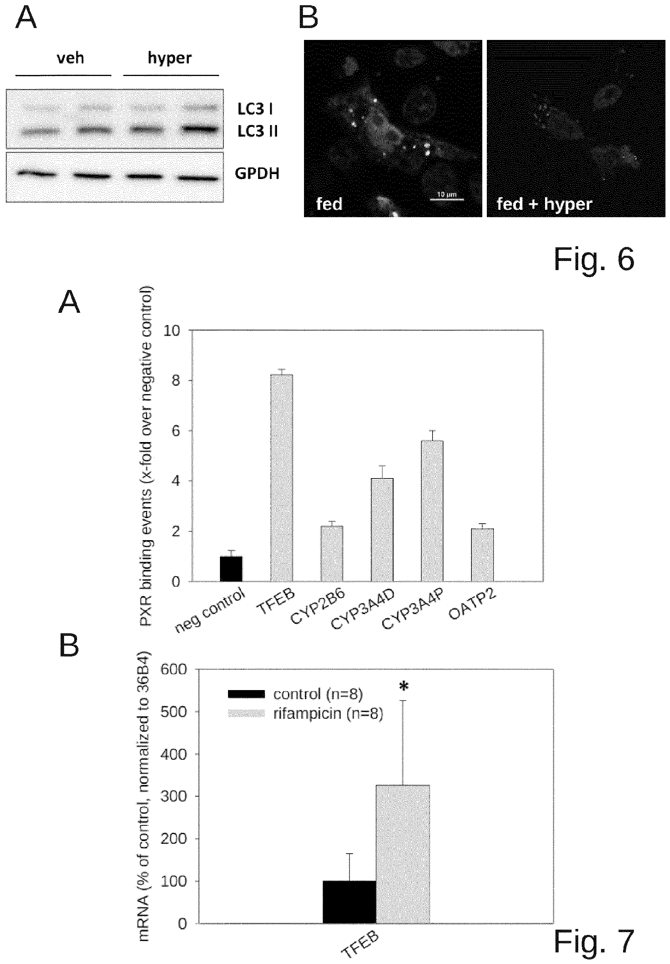

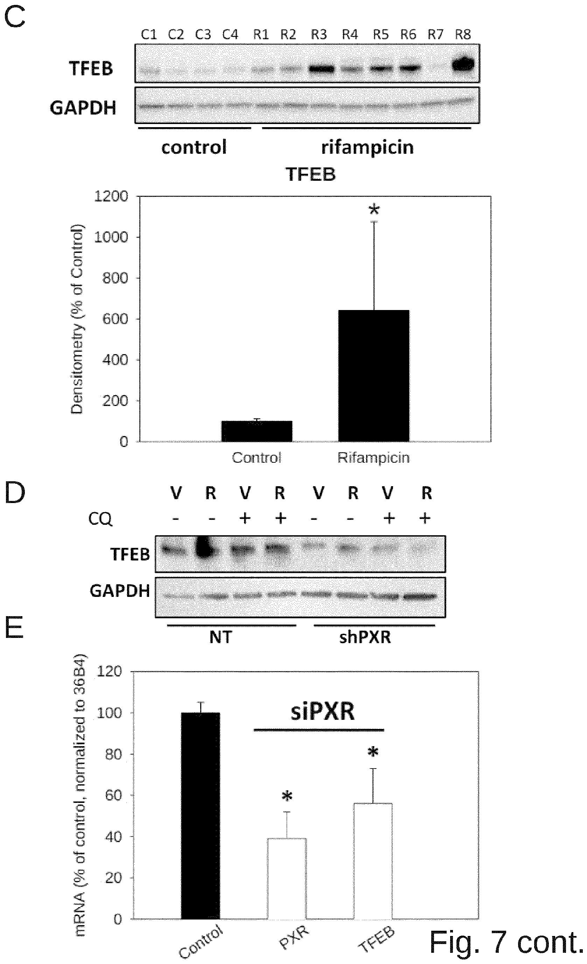

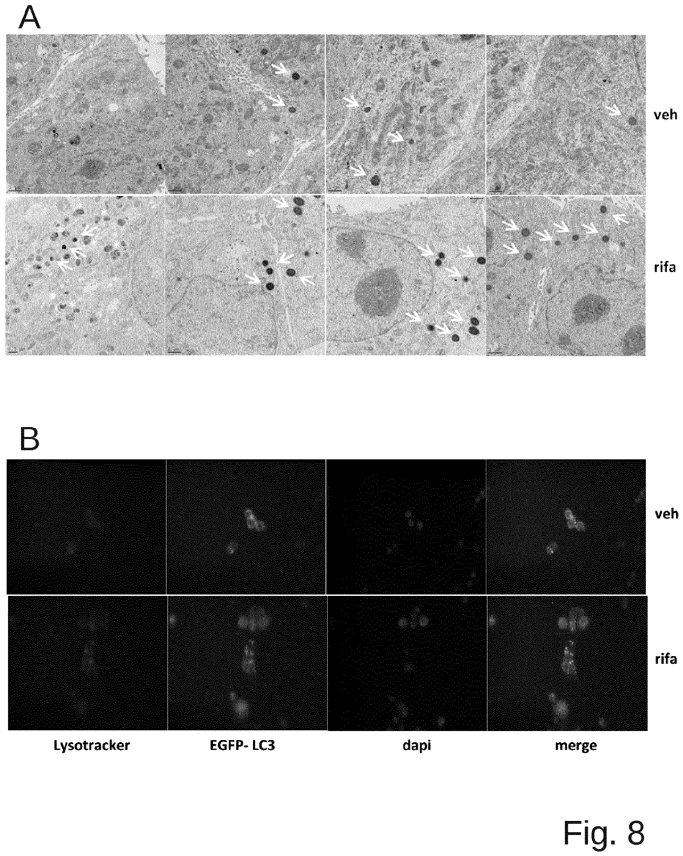

In the course of the present invention, it was demonstrated that PXR agonists such as rifampicin, hyperforin and UDCA activate TFEB via PXR (see Example 1 and Figures 7 - 9). Importantly, it was even shown that these ligands increase lysosomal bioformation (see Example 1 and Figure 8), making PXR agonists well suited for the treatment of LSDs.

TFEB targets the LIPA gene and therefore stimulation of TFEB leads to increased expression of LAL (see e.g. Dubland, Joshua A., and Gordon A. Francis. "Lysosomal acid lipase: at the cross-roads of normal and atherogenic cholesterol metabolism." Frontiers in cell and developmental biology 3 (2015): 3). In view of this teaching and upon having read the present description, the skilled person will appreciate that PXR agonists are thus particularly advantageous for the prevention or treatment of LAL-deficiency, including Wolman disease and CESD, among all lysosomal storage diseases.

In a preferred embodiment of the present invention, the metabolic disorder is A1AD.

In this context, the present invention also provides a combination therapy comprising a PXR ligand and A1AT. Such a combination treatment is particularly advantageous in the case of A1AD since A1AT augmentation therapy serves to provide A1AT to protect the patient's lung tissue from proteolytic damage, while the PXR ligand targets A1AT accumulation in the liver in order to prevent or treat A1AD associated liver disease.

It is therefore also preferred if the composition for use according to the invention further comprises alpha-1 antitrypsin (A1AT). In this case it is especially preferred if the composition is provided for intravenous administration, in particular as a powder for reconstitution (e.g. in a physiologic salt solution) or as a solution (e.g. an isotonic solution with physiological pH) .

A further preferred embodiment of the present invention relates to the composition for use according to the invention, wherein the PXR ligand is administered to a subject, and wherein A1AT is administered to the subject in a composition together with the PXR ligand, in combination with the PXR ligand, before the PXR ligand is administered, or after the PXR ligand has been administered. In this embodiment it is preferred if the A1AT is administered to the subject intravenously. It is further preferred if the PXR ligand is administered orally or intravenously.

In another preferred embodiment, the invention provides a combination therapy comprising a PXR ligand and a bronchodilator and/or a steroid, in particular for the treatment of A1AD. Bronchodilators and/or steroids are commonly used for the symptomatic treatment of lung disease associated with A1AD. The combination therapy according to the invention is particularly advantageous since the bronchodilators and/or steroids target the symptoms associated with lung-disease whereas the PXR ligand targets A1AT accumulation in the liver in order to prevent or treat A1AD-associated liver disease.

Bronchodilators and steroids are well known in the art (see e.g. Vogelmeier, Claus F., et al. "Global strategy for the diagnosis, management, and prevention of chronic obstructive lung disease 2017 report. GOLD executive summary." American journal of respiratory and critical care medicine 195.5 (2017): 557-582), although not in combination with the PXR ligands described herein. In the context of the present invention, the bronchodilator and/or steroid preferably is an active agent that is suitable for the treatment of chronic obstructive pulmonary disease. It is preferred if the steroid is a corticosteroid, in particular fluticasone, budesonide, beclomethasone, cicelsonide, or prednisolone. It is further preferred if the bronchodilator is a short-acting bronchodilator, in particular albuterol, levalbuterol, or ipratropium. In another preferred embodiment the bronchodilator is a long-acting bronchodilator, in particular aclidinium, arformoterol, formoterol, glycopyrrolate, indacaterol, olodaterol, salmeterol, tiotropium, or umeclidinium. In a further preferred embodiment combinations of bronchodilators and/or steroids are used, in particular the combination of a short-acting and a long-acting bronchodilator.

In another preferred embodiment of the invention, the inventive composition for use therefore further comprises at least one additional active agent selected from the group consisting of bronchodilators and steroids. In this case it is preferred if the composition is provided for inhalation, in particular as a powder or a solution.

A further preferred embodiment of the present invention relates to the composition for use according to the invention, wherein the PXR ligand is administered to a subject, and wherein a bronchodilator and/or a steroid is administered to the subject in a composition together with the PXR ligand, in combination with the PXR ligand, before the PXR ligand is administered, or after the PXR ligand has been administered. In this embodiment the bronchodilator and/or steroid is preferably administered by inhalation. The PXR ligand may e.g. be administered by inhalation, orally, or intravenously.

In a preferred embodiment of the present invention, the metabolic disorder is lysosomal storage disease (LSD). It is especially preferred if the LSD is LAL-deficiency, in particular Wolman disease or CESD.

In this context, the present invention also provides a combination therapy comprising a PXR ligand and sebelipase alfa. Such a combination is advantageous since the PXR ligand can increase endogenous LAL levels while sebelipase alfa provides additional recombinant LAL.

It is therefore also preferred if the composition for use according to the invention further comprises sebelipase alfa, preferably wherein the composition is provided for intravenous administration, preferably as a powder for reconstitution (e.g. in a physiologic salt solution) or as a solution (e.g. an isotonic solution with physiological pH).

A further preferred embodiment of the present invention relates to the composition for use according to the invention, wherein the PXR ligand is administered to a subject, and wherein sebelipase alfa is administered to the subject in a composition together with the PXR ligand, in combination with the PXR ligand, before the PXR ligand is administered, or after the PXR ligand has been administered. In this embodiment it is preferred if the sebelipase alfa is administered to the subject intravenously. It is further preferred if the PXR ligand is administered orally or intravenously.

In another embodiment, the PXR ligand is the single active agent in the pharmaceutical composition (preferably in the presence of one or more excipients).

The subject to be treated according to the present invention may be a human or a non-human animal. It is preferred if the subject is a mammal, most preferably a human. The subject preferably has or is predisposed to any one of the diseases and conditions mentioned herein.

The term "preventing" or "prevention" as used herein means to stop a disease state or condition from occurring in an individual completely or almost completely or at least to a (preferably significant) extent, especially when the individual is predisposed to such a risk of contracting a disease state or condition. However, these terms should not be interpreted as an absolute success in the sense that a subject can never develop an associated disease, reaction or condition but as the reduction of the chance of developing the disease, reaction or condition in a prophylactic treatment.

In the context of the present invention, the subject is preferably known to be at risk of developing A1AD or LSD, e.g. because of genetic risk factors, family history or other risk factors.

Typically, A1AD and LSDs are progressive disorders, implying that there is a period during which the disease has started, but definitive clinical symptoms sufficient for diagnosis have not yet appeared. The earlier phase of the disease in which no clinical symptoms are present but a disease-specific pathology (e.g. a genetic mutation) is present is typically referred to as the preclinical phase. During the preclinical phase, the disease can already be diagnosed, e.g. through genetic tests or other molecular tests.

The term "clinical symptom" as used herein, is to be understood as a manifestation of the disease that is apparent to the subject, e.g. shortness of breath, wheezing, persistent vomiting and diarrhea, or jaundice.

In a preferred embodiment of the invention, the A1AD or LSD is in the preclinical phase. According to a preferred embodiment, the subject does not yet present clinical symptoms associated with A1AD or LSD.

In a further embodiment, the subject has a (disease-causing) mutation in the SERPINA1 gene (National Center for Biotechnoglogy Information (NCBI) Entrez Gene GeneID 5265 for H. sapiens). Preferably the mutation is a mutation that causes A1AD, in particular a mutation as described in DeMeo, D. L., and E. K. Silverman. "α1-Antitrypsin deficiency· 2: Genetic aspects of α1-antitrypsin deficiency: phenotypes and genetic modifiers of emphysema risk." Thorax 59.3 (2004): 259-264. A1AT is encoded by the protease inhibitor (PI) locus located on chromosome 14q32.1. It is particularly preferred if the subject has the PI Z phenotype. The so-called PI Z phenotype is characterized by a Z pattern on serum isoelectric focusing and encompasses PI ZZ, PI MZ, PI SZ and PI Znull genotypes. In the context of the present invention, it is particularly preferred if the subject has the PI MZ, the PI SZ, or the PI ZZ genotype; especially the PI ZZ genotype.

In a further embodiment of the present invention, the subject has a mutation in the LIPA gene (NCBI Entrez Gene GeneID 3988 for H. sapiens). Preferably the mutation is a mutation that causes LAL-deficiency.

In a further preferred embodiment, the subject is a human, preferably a human having an age below 70 years, more preferably below 60 years, even more preferably below 50 years, yet even more preferably below 40 years and/or a human who had A1AD or LAL-deficiency onset before the age of 70 years, preferably before the age of 60 years, even more preferably below the age of 50 years, yet even more preferably below the age of 40 years.

In order to prevent or delay disease progression it is preferred if the treatment is continued for an extended period of time. Accordingly, in a preferred embodiment, the inventive composition, set or combination therapy is administered to the subject over a period of at least 2 weeks, preferably at least 4, more preferably at least 8, even more preferably at least 12, yet even more preferably at least 16 or even at least 20, especially at least 26 or even at least 52 weeks.

The present invention also relates to diagnosing a subject with a metabolic disorder selected from A1AD and lysosomal storage disease or a predisposition thereto and then treating the subject with a composition, set or combination therapy according to the present invention. The diagnosis is not necessarily performed together with the inventive treatment. Said diagnosis can e.g. be a genetic test.

The present invention also relates to a method for preventing or treating a metabolic disorder selected from A1AD and lysosomal storage disease in a subject comprising the steps of: (1) Identifying a subject having a metabolic disorder selected from A1AD and lysosomal storage disease or being at risk of developing a metabolic disorder selected from A1AD and lysosomal storage disease, preferably by a genetic test, in particular wherein the subject has a mutation in a gene associated with an increased risk of developing a metabolic disorder selected from A1AD and lysosomal storage disease, in particular wherein the gene is SERPINA1 or LIPA; (2) obtaining a PXR ligand selected from the group consisting of rifampicin, hyperforin and UDCA; and (3) administering an efficient amount of said PXR ligand to the subject.

In the context of the present invention, the expression "pharmaceutical composition" refers to any composition comprising at least one active agent (e.g. the PXR ligand), and preferably one or more excipients, which is pharmaceutically acceptable for administration to an individual, especially a mammal, in particular a human.

In a preferred embodiment, the composition or the composition for use according to the invention is provided for oral administration, preferably wherein the composition is provided as a tablet, a capsule, a suspension or a solution. If the pharmaceutical composition is intended for oral intake, it may also be in the form of pastilles, tablets, troches, lozenges, pills, gums, powders or drinking solutions. Systemic distribution of the PXR ligand can be facilitated by formulations and carriers known in the state of the art.

In a further preferred embodiment, the composition or the composition for use according to the invention is provided for intravenous administration, preferably wherein the composition is provided as a powder for reconstitution (e.g. in a physiologic salt solution) or as a solution (e.g. an isotonic solution with physiological pH).

In yet another preferred embodiment, the composition or the composition for use according to the invention is provided for inhalation, in particular as a powder or a solution. This embodiment is particularly preferred in combination with any embodiment, according to which the composition comprises bronchodilators and/or steroids.

The formulation or composition may comprise pharmaceutical carriers, excipients, preferably polymeric excipients, or additives. The term "carrier" refers to a diluent, e.g. water, saline, excipient, or vehicle, with which the composition can be administered. For a solid or fluid composition the carriers or additives in the pharmaceutical composition may comprise SiO₂, TiO₂, a binder, such as microcrystalline cellulose, polyvinylpyrrolidone (polyvidone or povidone), gum tragacanth, gelatine, starch, lactose or lactose monohydrate, alginic acid, maize (corn) starch and the like; a lubricant or surfactant, such as magnesium stearate, or sodium lauryl sulphate; a glidant, such as colloidal silicon dioxide; a sweetening agent, such as sucrose or saccharin. Preferably, the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. The PXR ligand can be in the form of a pharmaceutically acceptable salt, for example sodium salt of the PXR ligand. Other pharmaceutically acceptable salts include, among others, potassium, lithium and ammonium salts. Preferred excipients are polymers, especially cellulose and cellulose derivatives.

According to a particularly preferred embodiment of the present invention, the pharmaceutical composition is a liquid and preferably an aqueous solution. In general, liquid compositions are especially suitable for intravenous administration. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives, such as benzalkonium chloride. The pharmaceutical composition according to the present invention may be liquid or ready to be dissolved in liquid such as sterile, deionised or distilled water, or sterile isotonic phosphate-buffered saline (PBS). Preferably, 1000 µg (dry-weight) of such a composition comprises or consists of 0.1-990 µg, preferably 1-900µg, more preferably 10-200µg PXR ligand, and optionally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, deionised/distilled water or sterile isotonic phosphate-buffered saline (PBS) to yield a final volume of 0.1-100 ml, preferably 0.5-20 ml, more preferably 1-10 ml. Preferably, the concentration of the PXR ligand is 0.01 mM - 1000 mM, preferably 0.1 mM - 500 mM, more preferably 0.25 mM - 250 mM, even more preferably 0.5 mM - 100 mM, in particular 1 mM - 50 mM or even 2.5 mM - 25 mM.

It is evident to the skilled person that active agents and drugs described herein can also be administered in salt form (i.e. as a pharmaceutically acceptable salt of the active agent). Accordingly, any mention of an active agent herein shall also include any pharmaceutically acceptable salt forms thereof.

In a preferred embodiment, a dose of the composition is administered to a subject, preferably a human individual. It is preferred that the dose contains between 10 mg and 1000 mg, preferably between 50 and 1000 mg, more preferably between 100 mg and 1000 mg, even more preferably between 200 mg and 800 mg, most preferably between 400 mg and 600 mg, in particular 600 mg rifampicin. In another preferred embodiment, the dose is between 0.1 mg/kg body weight of a patient and 50 mg/kg, preferably between 0.4 mg/kg and 25 mg/kg, even more preferably between 2 mg/kg and 15 mg/kg, most preferably between 5 mg/kg and 10 mg/kg rifampicin. In a further preferred embodiment, a dose of the composition is administered to a subject, preferably a human individual, and wherein the dose contains between 0.05 mg and 500 mg, preferably between 0.2 and 200 mg, more preferably between 1 mg and 50 mg, even more preferably between 4 mg and 20 mg hyperforin. In another preferred embodiment, the dose is between 0.001 mg/kg body weight of a patient and 40 mg/kg, preferably between 0.01 mg/kg and 10 mg/kg, even more preferably between 0.05 mg/kg and 4 mg/kg, most preferably between 0.1 mg/kg and 1 mg/kg hyperforin. In yet another preferred embodiment, the dose contains between 10 mg and 10000 mg, preferably between 20 mg and 5000 mg, more preferably between 50 and 2000 mg, even more preferably between 100 mg and 1000 mg, yet even more preferably between 250 mg and 500 mg UDCA. In yet a further preferred embodiment, the dose is between 0.1 mg/kg body weight of a patient and 1000 mg/kg, preferably between 0.5 mg/kg and 200 mg/kg, even more preferably between 2 mg/kg and 50 mg/kg, most preferably between 10 mg/kg and 15 mg/kg UDCA.

It is further preferred, if the dose is administered at least once per week, preferably at least three times per week, even more preferably at least once per day, yet even more preferably at least twice per day, in particular three times per day.

The inhalation device according to the invention preferably is a metered-dose inhaler, a dry-powder inhaler or a nebuliser. A metered-dose inhaler is a device that aerosolises a predefined dose of a pharmaceutical composition (i.e. produces a comparatively short burst of aerosol with a defined dose), usually for self-administration by the patient. A metered-dose inhaler is for instance disclosed in US 6,260,549 B1. Dry-powder inhalers are devices for inhalation of dry-powder formulations by the patient. Such devices are for instance disclosed in US Patents 4,995,385 and 4,069,819. Established dry-powder inhalers are for instance SPINHALER®, ROTAHALER®, FLOWCAPS®, INHALATOR®, DISKHALER® and AEROLIZER®. Nebulisers are devices that produce aerosols for inhalation, typically continuously as long as they are switched on or breath-actuated. Established nebuliser products are for instance Aeroneb® and Pari®. Document US 9,364,618 B2, e.g., also discloses a nebuliser.

The present invention further relates to the following preferred embodiments:
Embodiment 1. A pharmaceutical composition for use in prevention or treatment of a metabolic disorder selected from alpha-1 antitrypsin deficiency (A1AD) and lysosomal storage disease, the composition comprising a pregnane X receptor (PXR) ligand selected from the group consisting of rifampicin, hyperforin and ursodeoxycholic acid (UDCA).
Embodiment 2. The composition for use according to embodiment 1, wherein the metabolic disorder is A1AD.
Embodiment 3. The composition for use according to embodiment 2, wherein the composition further comprises alpha-1 antitrypsin (A1AT), preferably wherein the composition is provided for intravenous administration, preferably as a powder for reconstitution or as a solution.
Embodiment 4. The composition for use according to any one of embodiments 2 or 3, wherein the composition further comprises at least one additional active agent selected from the group consisting of bronchodilators and steroids, preferably wherein the composition is provided for inhalation, preferably as a powder or a solution.
Embodiment 5. The composition for use according to any one of embodiments 2 to 4, wherein the PXR ligand is administered to a subject, and
   wherein A1AT is administered to the subject in a composition together with the PXR ligand, in combination with the PXR ligand, before the PXR ligand is administered, or after the PXR ligand has been administered.
Embodiment 6. The composition for use according to any one of embodiments 2 to 5, wherein the PXR ligand is administered to a subject, and
   wherein a bronchodilator and/or a steroid is administered to the subject in a composition together with the PXR ligand, in combination with the PXR ligand, before the PXR ligand is administered, or after the PXR ligand has been administered.
Embodiment 7. The composition for use according to any one of embodiments 1 to 6, wherein the subject is a human or a non-human animal.
Embodiment 8. A set comprising a first pharmaceutical composition comprising a PXR ligand selected from the group consisting of rifampicin, hyperforin and UDCA, and a second pharmaceutical composition comprising A1AT.
Embodiment 9. A set comprising a first pharmaceutical composition comprising a PXR ligand selected from the group consisting of rifampicin, hyperforin and UDCA, and a second pharmaceutical composition comprising a bronchodilator and/or a steroid.
Embodiment 10. The set according to embodiment 8 or 9, wherein the set is for prevention or treatment of a metabolic disorder selected from alpha-1 antitrypsin deficiency (A1AD) and lysosomal storage disease.
Embodiment 11. A pharmaceutical composition comprising A1AT and a PXR ligand selected from the group consisting of rifampicin, hyperforin and UDCA, preferably wherein the pharmaceutical composition is for intravenous administration, preferably wherein the pharmaceutical composition is a powder for reconstitution or a solution.
Embodiment 12. A pharmaceutical composition comprising an active agent selected from the group consisting of bronchodilators and steroids, and a PXR ligand selected from the group consisting of rifampicin, hyperforin and UDCA, preferably wherein the pharmaceutical composition is for inhalation, preferably wherein the pharmaceutical composition is a powder, a solution or an aerosol.
Embodiment 13. The pharmaceutical composition according to embodiment 11 or 12, wherein the pharmaceutical composition is for prevention or treatment of alpha-1 antitrypsin deficiency (A1AD).
Embodiment 14. An inhalation device for administration of a pharmaceutical composition, wherein the device comprises the pharmaceutical composition, wherein the composition comprises an active agent selected from the group consisting of bronchodilators and steroids, and a PXR ligand selected from the group consisting of rifampicin, hyperforin and UDCA; preferably wherein the inhalation device is a metered-dose inhaler, a dry-powder inhaler or a nebuliser, and/or preferably wherein the pharmaceutical composition is the pharmaceutical composition as defined in embodiment 12.
Embodiment 15. The inhalation device according to embodiment 14, wherein the inhalation device is for prevention or treatment of alpha-1 antitrypsin deficiency (A1AD).
Embodiment 16. The composition for use according to embodiment 1, wherein the metabolic disorder is lysosomal storage disease.
Embodiment 17. The composition for use according to embodiment 16, wherein the metabolic disorder is lysosomal acid lipase (LAL)-deficiency.
Embodiment 18. The composition for use according to embodiment 17, wherein the LAL deficiency is Wolman disease or Cholesteryl Ester Storage Disease (CESD).
Embodiment 19. The composition for use according to any one of embodiments 16 to 18, wherein the composition further comprises sebelipase alfa, preferably wherein the composition is provided for intravenous administration, preferably as a powder for reconstitution or as a solution.
Embodiment 20. The composition for use according to any one of embodiments 16 to 19, wherein the PXR ligand is administered to a subject, and
wherein sebelipase alfa is administered to the subject in a composition together with the PXR ligand, in combination with the PXR ligand, before the PXR ligand is administered, or after the PXR ligand has been administered.
Embodiment 21. The composition for use according to any one of embodiments 16 to 20, wherein the subject is a human or a non-human animal.
Embodiment 22. A set comprising a first pharmaceutical composition comprising a PXR ligand selected from the group consisting of rifampicin, hyperforin and UDCA, and a second pharmaceutical composition comprising sebelipase alfa.
Embodiment 23. The set according to embodiment 22, wherein the set is for prevention or treatment of alpha-1 antitrypsin deficiency (A1AD).
Embodiment 24. A pharmaceutical composition comprising sebelipase alfa and a PXR ligand selected from the group consisting of rifampicin, hyperforin and UDCA, preferably wherein the pharmaceutical composition is for intravenous administration, preferably wherein the pharmaceutical composition is a powder for reconstitution or a solution.
Embodiment 25. The pharmaceutical composition according to embodiment 24, wherein the pharmaceutical composition is for prevention or treatment of alpha-1 antitrypsin deficiency (A1AD).
Embodiment 26. A method for delaying the onset of or treating a metabolic disorder selected from A1AD and lysosomal storage disease, comprising
   - obtaining a pharmaceutically acceptable formulation comprising a PXR ligand selected from rifampicin, hyperforin and UDCA; and
   - administering an effective amount of the formulation to an individual having said metabolic disorder or at risk of developing said metabolic disorder.
Embodiment 27. The composition for use according to any one of embodiments 1 to 7, or 16 to 21, wherein a dose of the composition is administered to a subject, preferably a human individual, and wherein the dose contains between 0.1 mg/kg body weight of the subject and 50 mg/kg, preferably between 0.4 mg/kg and 25 mg/kg, even more preferably between 2 mg/kg and 15 mg/kg, most preferably between 5 mg/kg and 10 mg/kg rifampicin.
Embodiment 28. The composition for use according to any one of embodiments 1 to 7, 16 to 21, or 27, wherein a dose of the composition is administered to a subject, preferably a human individual, and wherein the dose contains between 0.001 mg/kg body weight of the subject and 40 mg/kg, preferably between 0.01 mg/kg and 10 mg/kg, even more preferably between 0.05 mg/kg and 4 mg/kg, most preferably between 0.1 mg/kg and 1 mg/kg hyperforin.
Embodiment 29. The composition for use according to any one of embodiments 1 to 7, 16 to 21, 27 or 28, wherein a dose of the composition is administered to a subject, preferably a human individual, and wherein the dose contains between 0.1 mg/kg body weight of the subject and 1000 mg/kg, preferably between 0.5 mg/kg and 200 mg/kg, even more preferably between 2 mg/kg and 50 mg/kg, most preferably between 10 mg/kg and 15 mg/kg UDCA.
Embodiment 30. The composition for use according to any one of embodiments 1 to 7, 16 to 21, or 27 to 29, wherein said dose is administered at least once per week, preferably at least three times per week, even more preferably at least once per day, yet even more preferably at least twice per day, in particular three times per day.

The present invention is further illustrated by the following figures and examples, without being restricted thereto.
**Figure 1****. The PXR ligand rifampicin induces markers of autophagy on protein and mRNA levels in human liver biopsy samples invivo.** Liver biopsy samples from individual patients receiving either placebo (control C1-C4, n=4) or rifampicin R1-R8, n=8) have been analyzed by Western blotting (A) and qPCR (B) for markers of autophagy. A. Rifampicin significantly induces the autophagy readout marker LC3 II. Densitometry of Western blot data for LC3 II are shown on the bottom. B. RT-qPCR of selected autophagy genes in control and rifampicin-treated patients (n=8 each) shows significant induction of ATG5, ATG10, LC3B/C and ATG16/1. * p<0.05, unpaired t-test.

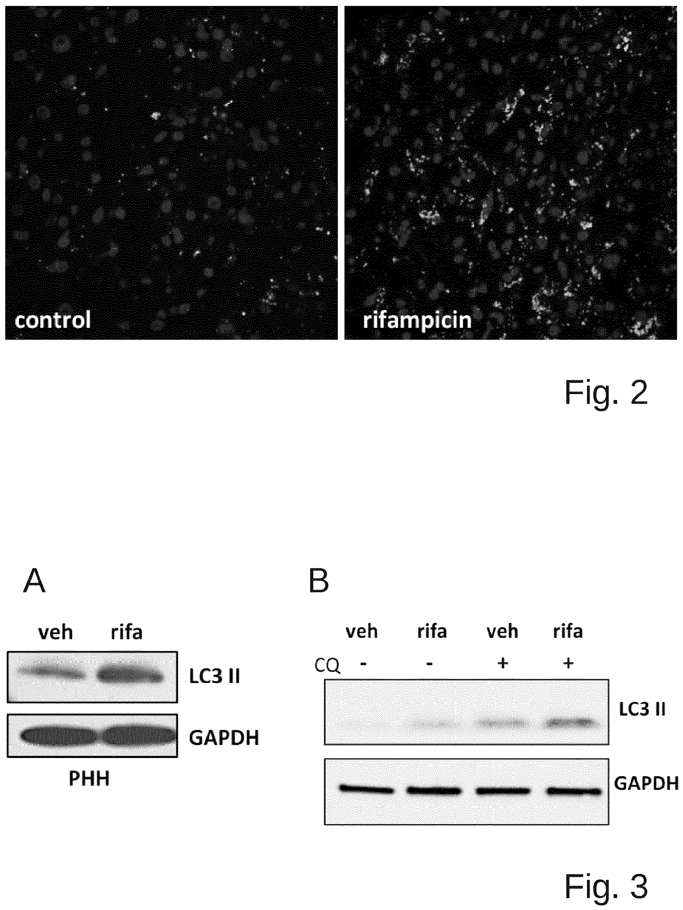

**Figure 2****. The PXR ligand rifampicin induces autophagic flux in human liver biopsy samples in vivo.** Liver biopsy samples from individual patients receiving either placebo (control) or rifampicin has been co-stained for dapi (blue, nuclei), LC3A (red, autophagosomes) and cathepsin D (green, lysosomes). Rifampicin significantly increases fusion of autophagosomes with lysosomes indicated by an increased merging orange signal. A representative picture is shown; magnification, 40x.

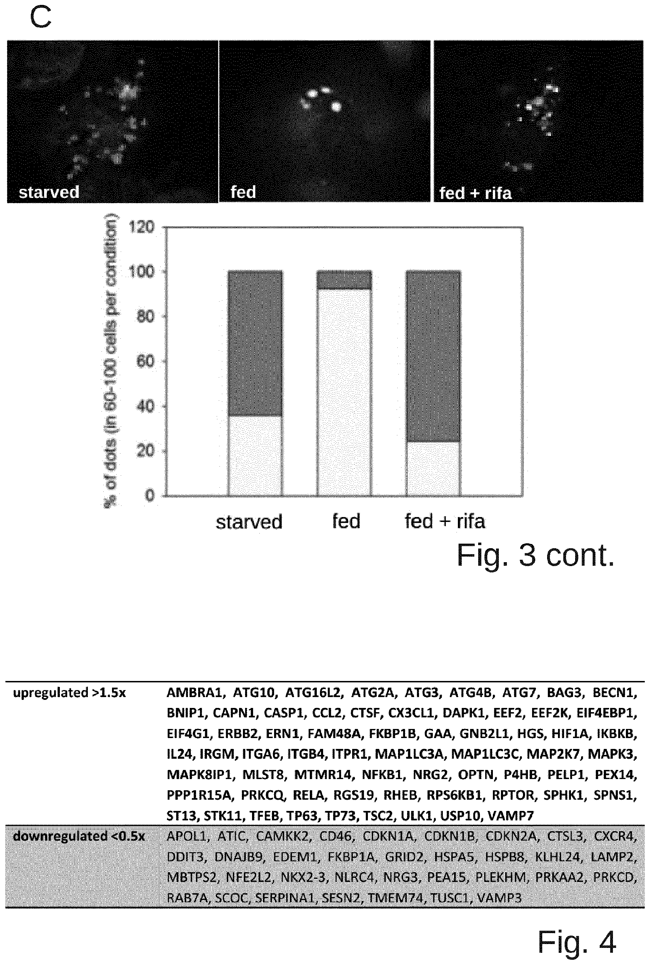

**Figure 3****. The PXR ligand rifampicin induces autophagic flux invitro.** Primary human hepatocytes (PHH) (A) and HepG2 cells (B, C) were treated with 50µM rifampicin (rifa) or ethanol as vehicle (veh). Autophagic flux assays was performed in HepG2 cells according the "Guidelines for the use and interpretation of assays for monitoring autophagy" (Klionsky DJ, Abdelmohsen K, Abe A, Abedin MJ, Abeliovich H, Acevedo Arozena A, Adachi H, et al. Guidelines for the use and interpretation of assays for monitoring autophagy (3rd edition). Autophagy 2016;12:1-222.). A. Rifampicin induces LC3 II protein of primary human hepatocytes (pooled duplicates of two different batches of PHH). B. Rifampicin induces LC3 II protein in the presence of the lysosomal inhibitor chloroquine (CQ) indicative of increased autophagic flux (pooled triplicates). Of note, in contrast to liver tissue, HepG2 cells display a prominent LC3 II band and only weak LC3 I. C. HepG2 cells were transfected with pGFP-RFP-LC3. Suppressed autophagy (fed condition) results in yellow dots as a merge of GFP and RFP fluorescence (middle). Induction of autophagy results in breakdown of GFP, resulting in red dots from the left-over RFP fluorescence indicating induced autophagic flux (starved condition) (left). Rifampicin increases autophagic flux in fed cells (right). Quantification by dot counting in 60-100 cells per condition (bottom panel).
**Figure 4****. Re-analysis of a RNA Seq data set focusing on autophagy-related genes.** Re-analysis of a published RNA Seq data set from rifampicin-treated human primary hepatocytes (Smith RP, et al. PLoS Genet 2014;10:e1004648). From a list of 231 autophagy-related genes 65 genes were upregulated > 1.5 fold and 34 genes downregulated < 0.5 fold.

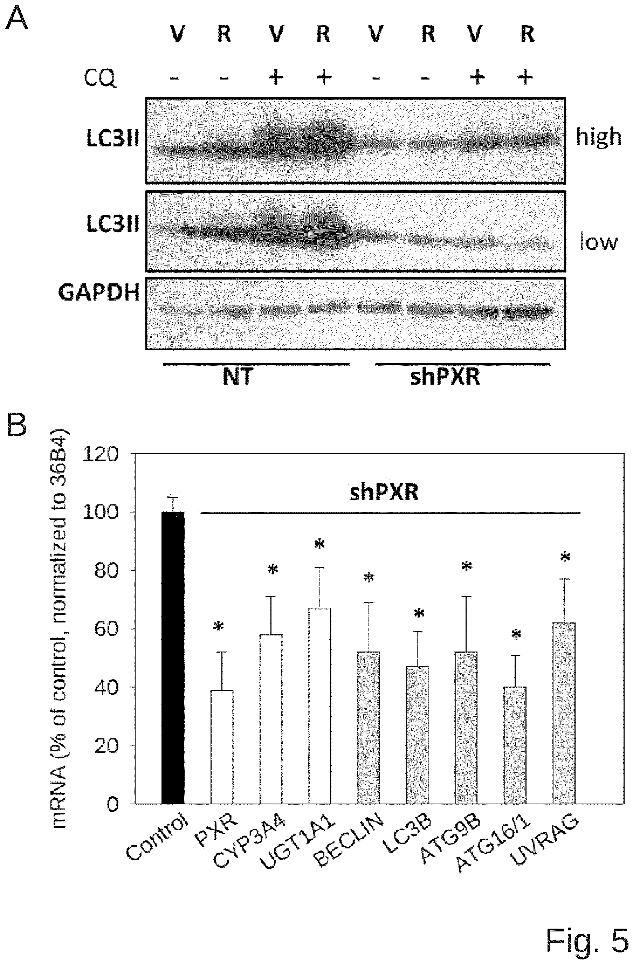

**Figure 5****. Rifampicin induces autophagy flux PXR dependently.** A. PXR was silenced using shRNA (shPXR) in HepG2 cells, non-targeting siOligos were used as control (NT). HepG2 cells were then treated with 50µM rifampicin (R) or ethanol as vehicle (V) in the absence or presence of the lysosomal inhibitor chloroquin (CQ). In shPXR HepG2 cells, chloroquine treatment leads to a markedly reduced accumulation of LC3 II (high and low exposure) indicating reduced basal autophagy. Induction of autophagic flux by rifampicin (R) is blocked in siPXR cells indicating PXR-dependent effects. B. mRNA expression levels of PXR, classical PXR target genes (white) and autophagy-related genes (gray) are significantly reduced in siPXR HepG2 cells compared to NT control HepG2 cells (black bar).
**Figure 6****. Hyperforin induces autophagy flux.** A. HepG2 cells were treated with 50µM hyperforin (hyper) or ethanol as vehicle (veh). Autophagic flux assays was performed in HepG2 cells according the "Guidelines for the use and interpretation of assays for monitoring autophagy" (Klionsky DJ, Abdelmohsen K, Abe A, Abedin MJ, Abeliovich H, Acevedo Arozena A, Adachi H, et al. Guidelines for the use and interpretation of assays for monitoring autophagy (3rd edition). Autophagy 2016;12:1-222.). A. Hyperforin induces LC3 II protein in HepG2 cells (pooled triplicates). C. HepG2 cells were transfected with pGFP-RFP-LC3. Suppressed autophagy (fed condition) results in yellow dots as a merge of GFP and RFP fluorescence (left). Induction of autophagy results in breakdown of GFP, resulting in red dots from the left-over RFP fluorescence indicating induced autophagic flux in hyperforin treated fed HepG2 cells (right).
**Figure 7****. TFEB is a direct PXR target and induced by rifampicin.** A. PXR binding site in the first intron of TFEB retrieved from PXR-ChIP Seq analysis and evaluated by PXR ChIP-PCR along with established PXR binding sites in a human liver biopsy invivo. B/C. Liver biopsy samples from individual patients receiving either placebo (control C1-C4, n=4) or rifampicin R1-R8, n=8) have been analyzed by qPCR and Western blotting for TFEB expression. Rifampicin significantly induces TFEB on mRNA (B) and protein levels (C) invivo. Densitometry of Western blot data for TFEB are shown on the bottom of (C). D/E. PXR was silenced using shRNA (shPXR) in HepG2 cells, non-targeting siOligos were used as control (NT). HepG2 cells were then treated with 50µM rifampicin (R) or ethanol as vehicle (V) in the absence or presence of the lysosomal inhibitor chloroquin (CQ). In shPXR HepG2 cells protein expression of TFEB (D) is significantly abolished and not inducible by rifampicin anymore. PXR knockdown also significantly reduces TFEB mRNA levels (E). * p<0.05, unpaired t-test.
**Figure 8****. Rifampicin increases lysosomal bioformation.** HepG2 cells were treated with 50µM rifampicin (rifa) or ethanol as vehicle (veh) and examined under fed conditions. A. Electronmicroscopy reveals significantly more lysosmes (arrows) and autophagolysomoses with breakdown products (asterixs) in rifampicin treated cells. B. Immunoflourescence with lysotracker (red, lysosmoses), transfected EGFP-LC3 (green, autophagosomes), dapi (blue, nuclei) and yellow merge. Rifampicin increases the number of lysosmomes which readily fuse with autophagosomes.
**Figure 9****. TFEB knockdown prevents LC3 induction by PXR ligands.** TFEB was silenced using siRNA (siTFEB) in HepG2 cells, non-targeting siOligos were used as control (NT). HepG2 cells were then treated with 50µM rifampicin (rifa), 50µM hyperforin (hyper) or ethanol as vehicle (veh) in the absence or presence of the lysosomal inhibitor chloroquin (CQ). A. Rifampicin induces LC3 II in the presence of TFEB. LC3 II induction is blunted in siTFEB cells indicating TFEB dependent autophagy induction. B. Hyperforin induces LC3 II in the presence of TFEB. LC3 II induction is blunted in siTFEB cells indicating TFEB dependent autophagy induction.
**Figure 10****. Rifampicin and hyperforin reduce pathological alpha-1-antitrypsin accumulation.** HeLa cells were transfected with PXR/RXR and an empty plasmid (EV) or a plasmid containing either the normal (M) or the pathological Z-allel (Z) of alpha-1-antitrypsin leading to pathological protein accumulation. Cells were then treated for 24h with vehicle (ethanol), 50µM rifampicin or 50µM hyperforin. Rifampicin and hyperforin treatment robustly decreases pathological A1AT accumulation.

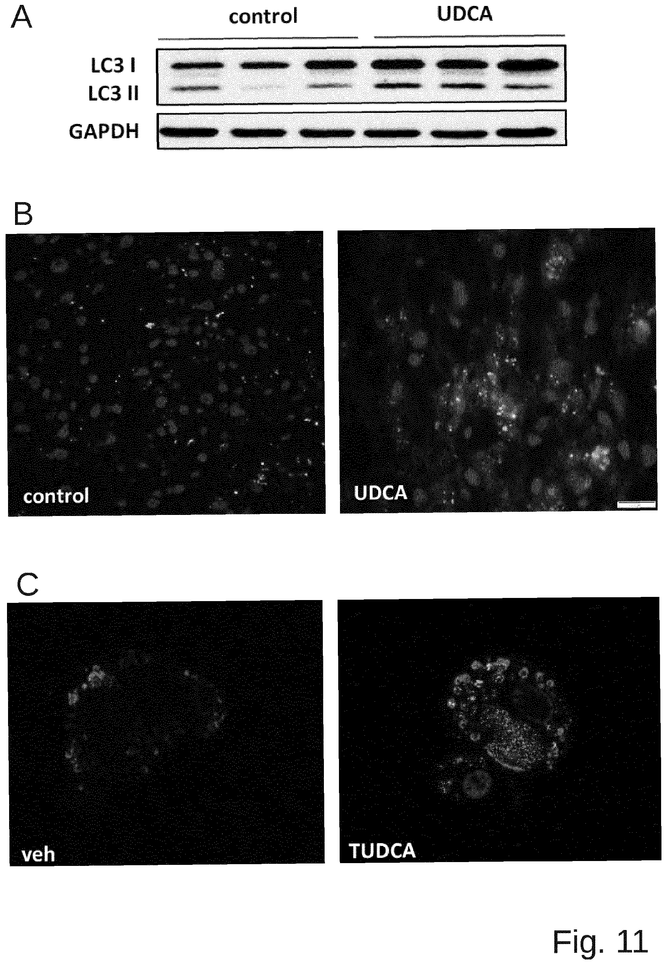

**Figure 11****. UDCA induces autophagy in vivo and in vitro.** A. Liver biopsy samples from individual patients receiving either placebo (control) or UDCA have been analyzed by Western blotting. UDCA significantly induces the autophagy readout marker LC3 II. B. A representative liver biopsy sample from a patient receiving placebo (control) or UDCA has been co-stained for dapi (blue, nuclei), LC3A (red, autophagosomes) and cathepsin D (green, lysosomes). UDCA significantly increases fusion of autophagosomes with lysosomes indicated by an increased merging orange signal. magnification, 40x. C. HepG2 cells were transfected with pEGFP-LC3 (green, autophagosomes) and co-stained with LAMP1 (red, lysosomes). 100µM Tauro-UDCA (TUDCA) induces autophagogomes and autophagosmal-to-lysosomal fusion.

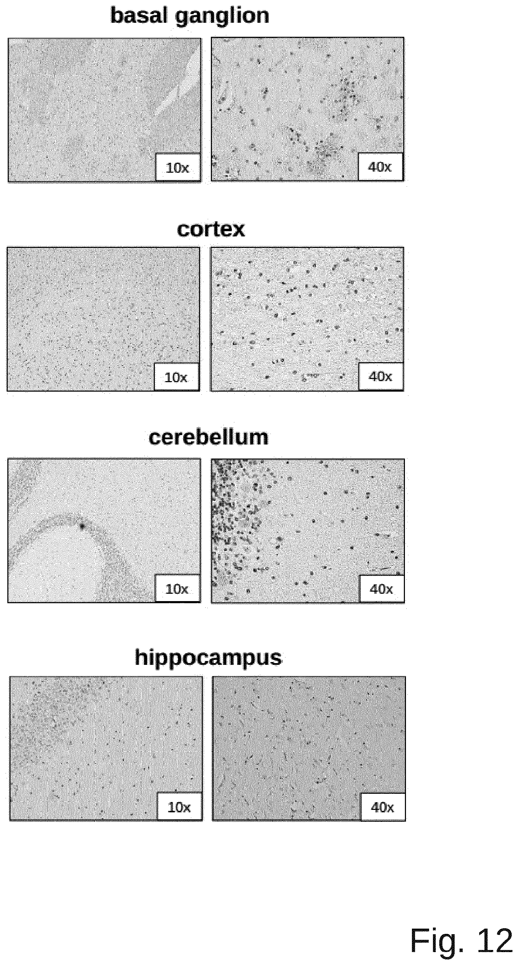

**Figure 12****. PXR is expressed in the human brain.** Tissue sections from basal ganglion, cortex, cerebellum, and the hippocampus. Immunohistochemical staining for PXR confirmed that PXR is expressed in neurons. Therefore, it is highly plausible that autophagy can be stimulated in the brain by activating PXR. Representative pictures are shown; magnification, 10x or 40x as indicated.

### Example 1 - The PXR-TFEB-autophagy signalling pathway is a new therapeutic drug target for the treatment of metabolic diseases including A1AD and lysosomal storage

### Materials and Methods

**Cell culture and transfection:** Cells were kept under regular growth conditions (5% CO₂) in DMEM media containing 10% FBS, 1% Penicillin/Streptomycin and 1/100 Na-Pyruvate (all Gibco). Prior to transfections, cells were plated in 6-well dishes at 2x10^5 cells/well. Cells were transfected with GFP-RFP-LC3 (gift from David D Moore, Baylor College of Medicine, Houston, TX, USA) or EGFP-LC3 (Addgene, Watertown, USA) using Lipofectamine 3000 as transfectant as per protocol (Invitrogen). Lysotracker (Thermo Fisher) was used to stain lysosomes according to protocol. After 24h the transfection media was changed to fresh media containing the respective treatment. Cells were treated with either chloroquine[25µM], rifampicin[50µM], hyperforin[1µM], tauro-ursodeoxycholic acid (TUDCA[100µM]) or Vehicle (Ethanol) for another 24h. After that cells were harvested and processed for further investigation.
   For siRNA experiments the respective siRNA nucleotides were purchased from Dharmacon (smartpool) and transfected with RNAiMaX (Invitrogen) as per protocol. For shPXR HepG2 cells were transduced with attenuated adenovirus (titer: 10^5/6-well) produced in 293T cells. The respective plasmids were a kind gift of David D Moore, Baylor College of Medicine, Houston, TX, USA.
**RNA preparation, RT qPCR and ChIP PCR:** RNA isolation from human tissue (Marschall HU, Wagner M, Zollner G, Fickert P, Diczfalusy U, Gumhold J, Silbert D, et al. Complementary stimulation of hepatobiliary transport and detoxification systems by rifampicin and ursodeoxycholic acid in humans. Gastroenterology 2005;129:476-485.) and cell lines was performed following the RNeasy protocol (Qiagen, RNeasy Mini kit) including DNAse I digest on column. RNA concentrations were measured by Nanodrop and 1000ng of RNA were subjected to cDNA synthesis (Superscript III, Invitrogen). RT qPCR was performed on a Light cycler (Roche) with Luna Universal qPCR Master Mix (NEB). All RT qPCR data was normalized to the house keeping gene 36B4 and the fold change was calculated with the ΔΔCt method. Ct values >35 were considered as negative.
   For ChIP PCR, a ChIP preparation following the Active motif high sensitivity kit's protocol was performed. 3 different binding sites were tested in normal liver tissue based on an in silico PXR response element search. 1 site was significantly occupied by TFEB. This site is located app. 7kb upstream of the first exon of the short variant of TFEB or otherwise in the first intron of the long variant of TFEB in an H3K27Ac active site (UCSC).
**Western blot:** Protein lysate was generated by homogenization (Magnalyzer, Roche) of tissue or cell pellet in homogenization buffer with protease and phosphatase inhibitors (Halt tablets, Invitrogen). After homogenization, lysates were sonicated 2x for 10" each. Lysates were then centrifuged for 10 min at 4°C and the supernatant was subjected to BCA protein estimation (Thermo Fisher). Equal amounts of lysate were loaded to self-made acrylamide gels. After separation, the protein was transferred to a PVDF membrane with semi-dry transfer. Primary antibody incubation (LC3B, Novus; GAPDH, Santa Cruz; TFEB, Abcam) took place over night at 4°C and secondary antibody (anti-rabbit-HRP or anti mouse-HRP) incubation for 1.5-2 h at room temperature. Development of the blots was performed with Clarity reagent (Biorad) on a Biorad developer. For native gels homogenization took place through 1 freeze thaw cycle in homogenization buffer with protease and phosphatase inhibitors (Halt tablets, Invitrogen). All buffers, dyes and gels were prepared without SDS.
**Immunofluorescence (IF) and Immunohistochemistry (IHC):** For IHC tissue sections (4µM for cryo, 2µM for paraffin) were cut and processed as per the antibody supplier's protocol for IHC. Briefly, sections were fixed in 4% paraformaldehyde (PFA), antigen retrieval was performed and then the sections were incubated with the primary (LC3A, Cell Signaling; Cadhepsin D, Abcam) and secondary antibody (anti-rabbit Alexa Fluor 488 or anti mouse Alexa Fluor 594) sequentially for 1h at room temperature. Slides were developed with rabbit-on-rodent-HRP-polymer (Biocare Medical). For IF cells were grown on glass chamber slides. They were fixed in 4% PFA, incubated with the primary and secondary antibody sequentially for 1h at room temperature followed by the secondary antibodies in the same way. Slides were mounted in DAPI containing mounting media (Invitrogen) and kept in the dark. Microscopy was performed on a Nikon laser scan microscope.
**Electron Microscopy (EM):** EM was performed using standard protocols. Treated cells fixed in 2% PFA + 2,5% glutaraldehyde in 0.1M NA-phosphate buffer were provided as starting material.
**ChIP-seq re-evaluation:** An already published dataset of Rifampicin treated primary human hepatocytes (Smith RP, Eckalbar WL, Morrissey KM, Luizon MR, Hoffmann TJ, Sun X, Jones SL, et al. Genome-wide discovery of drug-dependent human liver regulatory elements. PLoS Genet 2014;10:e1004648; Bioprojects PRJNA 239635 and 239637) was re-analyzed specifically for its TFEB expression using the galaxy platform for various analyses (https://galaxy.medunigraz.at).

### Results:

In a previous study we have treated patients with the human PXR ligand rifampicin to test whether "classical" phase I, phase II and phase III detoxifying enzymes/transporters are induced in human liver tissue (Marschall HU, et al. Gastroenterology 2005;129:476-485). We now showed that in livers of rifampicin-treated patients LC3 (microtubule-associated protein 1A/1B-light chain 3) II, as a readout marker for autophagy, is significantly increased on protein levels along with several autophagy-related genes (i.e. ATG5, ATG10, LC3B and LC3C) on RNA levels (Figure 1A and 1B). Rifampicin increased autophagy flux in human liver, since autophagosomes and lysosomes increasingly fused to autoph-agolysosomes under treatment conditions (Figure 2). The effects of autophagy induction by rifampicin were reproduced in cell culture experiments with primary human hepatocytes (Figure 3A) and the liver cell line HepG2 (Figure 3B and 3C) and confirmed increased autophagic flux in co-treatment assays with chloroquine (Figure 3B) and LC3-GFP-RFP co-labeling experiments (Figure 3C). In addition, when re-analyzing a published RNA Seq data set from rifampicin-treated human primary hepatocytes (Smith RP, et al. PLoS Genet 2014;10:e1004648), we found from a list of 231 autophagy-related genes 65 genes upregulated >1.5 fold and 34 genes downregulated <0.5 fold (Figure 4). To test if the observed effects truly depend on PXR we next knocked-down PXR in human hepatocytes and treated cells with rifampicin +/- chloroquine (the latter one commonly used to block autophagic flux (Klionsky DJ, Abdelmohsen K, Abe A, Abedin MJ, Abeliovich H, Acevedo Arozena A, Adachi H, et al. Guidelines for the use and interpretation of assays for monitoring autophagy (3rd edition). Autophagy 2016;12:1-222)). Accumulation of LC3 was significantly impaired already under baseline conditions when autophagic flux was blocked by chloroquine. Moreover, the rifampicin induced increase of autophagy was completely blunted in PXR knockdown cells (Figure 5A). On transcriptional levels, autophagy-related genes such as ATG5, ATG10, RAB7 (not shown), BECLIN, LC3B, ATG9B were significantly reduced in PXR knockdown cells (Figure 5B). Similar effects of autophagy induction were observed with hyperforin, the main active compound of St. John's wort (Figure 6). Of note, we also tested whether activation of the other xenobiotic receptor CAR has any impact on autophagy (using our RFP-GFP-LC3 cell line assay) but could not detect any changes in autophagy activity when cells were treated with the specific human CAR ligand CITCO (not shown).

Mechanistically, the master regulator for lysosomal biogenesis, transcription factor EB (TFEB) (Settembre C, Di Malta C, Polito VA, Garcia Arencibia M, Vetrini F, Erdin S, Erdin SU, et al. TFEB links autophagy to lysosomal biogenesis. Science 2011;332:1429-1433) is a key mediator of the PXR mediated regulation of autophagy. Re-analysis of a published PXR ChIP-Seq data set (Smith RP, et al. PLoS Genet 2014;10:e1004648) showed a robust PXR binding site in the first intron of TFEB, which we could re-capitulate by PXR ChIP PCR in human liver (Figure 7A). Moreover, TFEB was also significantly induced in our human biopsy samples on protein and mRNA levels (Figure 7B and 7C) and vice versa downregulated in the shPXR HepG2 cells on protein and transcript levels (Figure 7D and 7E). In line with the functional properties of TFEB lysosomes were elevated in numbers as demonstrated with electron microscopy (Figure 8A) and lysosomal IF staining (Figure 8B). Most convincingly, knockdown of TFEB significantly abrogated LC3 induction by rifampicin and hyperforin (Figure 9).

Functionally, we tested invitro whether rifampicin and hyperforin are able to reduce aggregates of pathological alpha-1-antitrypsin which typically accumulate in alpha-1-antitrypsin related chronic liver disease. Both, rifampicin and hyperforin robustly reduced pathological alpha-1-antitrypsin aggregates invitro in a human cell line (Figure 10) making it highly plausible that PXR ligands are well suited for the treatment of A1AD.

In addition to the autophagy inducing effects of PXR ligands we also observed a robust autophagy increase with the non-toxic bile acid ursodeoxycholic acid (UDCA) which has been clinically used for almost 50 years to treat gallstone disease and chronic cholestatic disorders. Patients treated with UDCA (Marschall HU, et al. Gastroenterology 2005;129:476-485) show a significant increase of LC3 II protein along with immunohistochemical autophagolysosome formation. In vitro UDCA treatment confirms the significantly increased autophagolysosome formation (Figure 11).

## Claims

1. A pharmaceutical composition for use in prevention or treatment of a metabolic disorder selected from alpha-1 antitrypsin deficiency (A1AD) and lysosomal storage disease, the composition comprising a pregnane X receptor (PXR) ligand selected from the group consisting of rifampicin, hyperforin and ursodeoxycholic acid (UDCA).

2. The composition for use according to claim 1, wherein the metabolic disorder is A1AD.

3. The composition for use according to claims 2, wherein the PXR ligand is administered to a subject, and
wherein alpha-1 antitrypsin (A1AT) is administered to the subject in a composition together with the PXR ligand, in combination with the PXR ligand, before the PXR ligand is administered, or after the PXR ligand has been administered.

4. The composition for use according to claims 2 or 3, wherein the PXR ligand is administered to a subject, and
wherein a bronchodilator and/or a steroid is administered to the subject in a composition together with the PXR ligand, in combination with the PXR ligand, before the PXR ligand is administered, or after the PXR ligand has been administered.

5. The composition for use according to claim 1, wherein the metabolic disorder is lysosomal storage disease.

6. The composition for use according to claim 5, wherein the metabolic disorder is lysosomal acid lipase (LAL)-deficiency.

7. The composition for use according to claim 5 or 6, wherein the PXR ligand is administered to a subject, and
wherein sebelipase alfa is administered to the subject in a composition together with the PXR ligand, in combination with the PXR ligand, before the PXR ligand is administered, or after the PXR ligand has been administered.

8. The composition for use according to any one of claims 1 to 7, wherein the subject is a human or a non-human animal.

9. A pharmaceutical composition comprising A1AT and a PXR ligand selected from the group consisting of rifampicin, hyperforin and UDCA, preferably wherein the pharmaceutical composition is for intravenous administration, preferably wherein the pharmaceutical composition is a powder for reconstitution or a solution.

10. A pharmaceutical composition comprising an active agent selected from the group consisting of bronchodilators and steroids, and a PXR ligand selected from the group consisting of rifampicin, hyperforin and UDCA, preferably wherein the pharmaceutical composition is for inhalation, preferably wherein the pharmaceutical composition is a powder, a solution or an aerosol.

11. A pharmaceutical composition comprising sebelipase alfa and a PXR ligand selected from the group consisting of rifampicin, hyperforin and UDCA, preferably wherein the pharmaceutical composition is for intravenous administration, preferably wherein the pharmaceutical composition is a powder for reconstitution or a solution.

12. An inhalation device for administration of a pharmaceutical composition, wherein the device comprises the pharmaceutical composition, wherein the composition comprises an active agent selected from the group consisting of bronchodilators and steroids, and a PXR ligand selected from the group consisting of rifampicin, hyperforin and UDCA; preferably wherein the inhalation device is a metered-dose inhaler, a dry-powder inhaler or a nebuliser, and/or preferably wherein the pharmaceutical composition is the pharmaceutical composition as defined in claim 10.

13. A set comprising a first pharmaceutical composition comprising a PXR ligand selected from the group consisting of rifampicin, hyperforin and UDCA, and a second pharmaceutical composition comprising A1AT.

14. A set comprising a first pharmaceutical composition comprising a PXR ligand selected from the group consisting of rifampicin, hyperforin and UDCA, and a second pharmaceutical composition comprising a bronchodilator and/or a steroid.

15. A set comprising a first pharmaceutical composition comprising a PXR ligand selected from the group consisting of rifampicin, hyperforin and UDCA, and a second pharmaceutical composition comprising sebelipase alfa.
